# EUROPEAN PATENT SPECIFICATION

(11) **EP 1 068 011 B1**
(45) Date of publication and mention of the grant of the patent: **18.08.2004**
(21) Application number: 99917966.6
(22) Date of filing: 29.03.1999
(51) Int. Cl.: B01J 31/40, C07D 301/12

(54) **PROCESS FOR THE EPOXIDATION OF PROPENE**
VERFAHREN ZUR EPOXIDIERUNG VON PROPEN
PROCEDE POUR L'EPOXYDATION DE PROPENE

(30) Priority: 30.03.1998 EP 98201002
(43) Date of publication of application: 17.01.2001
(73) Proprietor: SHELL INTERNATIONALE RESEARCH MAATSCHAPPIJ B.V., 2596 HR Den Haag (NL)
(72) Inventor: CROCKER, Mark, NL-1031 CM Amsterdam (NL); DERKS, Willem, NL-1031 CM Amsterdam (NL); DIRKZWAGER, Hendrik, NL-1031 CM Amsterdam (NL); HEROLD, Rudolf, Henri, Max, NL-1031 CM Amsterdam (NL); NIELE, Franciscus, Gerardus, Maria, NL-1031 CM Amsterdam (NL); WERMELING, Rutger, Johannes, Franciscus, NL-4782 SJ Moerdijk (NL)
(86) International application number: PCT/EP1999/002369
(87) International publication number: WO 1999/049972

(56) References cited:
- EP-A- 0 712 852
- US-A- 3 696 025
- US-A- 5 620 935

## Description

The present invention relates to a process for the epoxidation, of propene into propylene oxide.

A commonly known method for manufacturing propylene oxide is the coproduction of propylene oxide and styrene starting from ethylbenzene. In general such process involves the steps of (i) reacting ethylbenzene with oxygen or air to form ethylbenzene hydroperoxide, (ii) reacting the ethylbenzene hydroperoxide thus obtained with propene in the presence of an epoxidation catalyst to yield propylene oxide and 1-phenyl ethanol, and (iii) converting the 1-phenyl ethanol into styrene by dehydration using a suitable dehydration catalyst.

Another method for producing propylene oxide is the coproduction of propylene oxide and methyl tert-butyl ether (MTBE) starting from isobutane and propene. This process is well known in the art and involves similar reaction steps as the styrene/propylene oxide production process described in the previous paragraph. In the epoxidation step tert-butyl hydroperoxide is reacted with propene forming propylene oxide and tert-butanol. Tert-butanol is subsequently etherified into MTBE.

The present invention concerns the epoxidation step, and more in particular the epoxidation catalyst used therein. As indicated herein before the epoxidation step may involve the epoxidation reaction of propene with ethylbenzene hydroperoxide into propylene oxide and 1-phenyl ethanol or the epoxidation reaction of propene with tert-butyl hydroperoxide to yield propylene oxide and tert-butanol. The present invention will be further discussed herein on the basis of the epoxidation step in a styrene/propylene oxide coproduction process. It will, however, be understood that the invention can equally suitably be applied in the epoxidation step of an MTBE/propylene oxide coproduction process.

Titanium-containing heterogeneous epoxidation catalysts are known in the art. Examples of such catalysts are for instance described in US-A-4,367,342 and EP-A-0,345,856. US-A-4,367,342 discloses the use of inorganic oxygen compounds of silicon in chemical composition with at least 0.1% by weight of an oxide or hydroxide of titanium, while EP-A-0,345,856 discloses a titanium/silica heterogeneous catalyst. According to EP-A-0,345,856 this catalyst is obtainable by impregnating a silicon compound with a stream of gaseous titanium tetrachloride followed by calcination and hydrolysis steps and optionally a silylation step.

When such titanium-containing heterogeneous catalysts are used to catalyse the epoxidation of propene, deactivation occurs. Without any preventive measures a titanium-containing catalyst, which is contacted with a stream containing propene and ethylbenzene hydroperoxide from the preceding oxidation step, will have a limited lifetime before it will have to be replaced due to deactivation. In commercial operation deactivation of the catalyst is counteracted by operating at an increased reaction temperature. However, a too high a reaction temperature, for instance above 140 °C, cannot be afforded, as the reaction selectivity to propylene oxide deteriorates at such high reaction temperatures. Furthermore, high reaction temperatures favour the formation of byproducts, such as methyl phenyl ketone (decomposition product of ethylbenzene hydroperoxide), propionaldehyde (isomerisation product of propylene oxide) and other derivatives of propylene oxide including propylene glycol and polyols. Consequently, if the temperature becomes unacceptably high, the catalyst has to be replaced by a fresh or regenerated catalyst, thus enabling to operate at lower reaction temperatures, practically between 50 and 125 °C.

An increase of the lifetime of the catalyst would be beneficial, as it would result in a higher and more cost effective production of propylene oxide. It would furthermore reduce the costs due to catalyst consumption and the time and costs involved in reloading of the reactors.

The present invention provides a process for activating an at least partly deactivated, heterogeneous, titanium-containing epoxidation catalyst by making use of a soluble titanium compound. The process according to the present invention results in an increased lifetime of the catalyst and hence in a more cost effective and productive process.

Accordingly, the present invention relates to a process for the epoxidation of propene into propylene oxide, which process comprises adding a titanium compound to the liquid feed of an epoxidation reaction before this feed is contacted with the at least partly deactivated heterogeneous titanium containing catalyst inside the reactor, wherein the solubility of the titanium compound in the liquid feed is higher than the amount of titanium compound added to this feed.

The heterogeneous titanium-containing catalyst may be any such catalyst known in the art to be suitable for catalysing the reaction between an olefin and an organic peroxide into the corresponding alkylene oxide and alcohol. Accordingly, the catalysts disclosed in the patent specifications US-A-4,367,342 and EP-A-0,345,856 discussed above may, for instance, be applied. It has, however, been found particularly advantageous to use the titanium/silica based catalysts disclosed in EP-A-0,345,856 for the purpose of the present invention. When these catalysts are used, very good activation results can be achieved.

The composition of the feed to the epoxidation reaction is not critical for the process of the present invention in the sense that it may have any composition which is common in commercial operation. Accordingly, in case of a styrene/propylene oxide coproduction process, it comprises at least some ethylbenzene hydroperoxide (EBHP) and normally also some ethylbenzene. Propene is either added to the reactor as a separate feed stream or may be added to the EBHP-containing feed stream prior to entry into the epoxidation reactor. The feed may also contain some methyl phenyl ketone and/or 1-phenyl ethanol formed in the preceding oxidation section or in a preceding epoxidation reactor or contained in a recycle stream. A typical feed stream to the epoxidation reactor, which is first in line after the preceding oxidation step, comprises 15-25 wt% EBHP, 30-50 wt% ethylbenzene, 30-45 wt% propene, 0-5 wt% 1-phenyl ethanol and 0-5 wt% methyl phenyl ketone, to a total of 100 wt%.

In a MTBE/propylene oxide coproduction process the feed to the epoxidation reactor comprises at least some tert-butyl hydroperoxide (TBHP) in a tert-butanol solvent. Similar as in the styrene/propylene oxide coproduction process, propene is either added to the reactor as a separate feed stream or may be added to the TBHP-containing feed stream prior to entry into the epoxidation reactor.

In a commercial styrene/propylene oxide coproduction process heterogeneously catalysed epoxidation is typically carried out in a series of fixed bed reactors with intermediate cooling. This mode of operation is also suitably applied for the purpose of the present invention. The conditions under which the epoxidation reaction is carried out are those conventionally applied in propene epoxidation reactions with EBHP. Typical reaction conditions include temperatures of 50 to 140 °C, suitably 75 to 125 °C, and pressures up to 80 bar with the reaction medium being in the liquid phase.

The amount in which the titanium compound is added to the epoxidation feed may vary between broad limits. In any event the amount added to the feed must be less than the solubility of the titanium compound in that feed, so that the compound is completely dissolved in the feed. The amount added should be such that at least some activation effect can be noticed, while on the other hand the amount added should not be too high for environmental and economic reasons. Accordingly, it has been found practical to add the titanium compound in such amount that the titanium content (as metal) in the feed entering the epoxidation reactor is in the range of from 1 to 100 ppmw, preferably from 5 to 50 ppmw, based on total feed. Most preferably the amount added lies in the range of from 5 to 20 ppmw. The abbreviation "ppmw" refers to parts per million on a parts by weight basis.

Suitable titanium compounds are those titanium compounds which are soluble in the feed and remain dissolved in the reaction mixture inside the reactor. Preferably, the titanium compound is an organic titanium compound. Of the class of soluble organic titanium compounds, the organic titanium oxide compounds or organic titanates are most preferably applied.

One class of suitable titanium compounds is formed by the titanasilsesquioxane compounds disclosed in WO-A-97/24344. Accordingly, suitable titanium compounds for the purpose of the present invention include titanasilsesquioxane compounds of the general formula

TiLR₇Si₇O₁₂ (I)

wherein L is an alkyl, cycloalkyl, arylalkyl, alkylaryl, alkoxy, aryloxy, siloxy, amido or hydroxyl group and R is a cyclopentyl, cyclohexyl or cycloheptyl group. Preferred titanasilsesquioxane compounds are those compounds of the above formula, wherein L is chosen from phenoxy, isopropoxy, benzyl, trimethylsiloxy and dimethyl amido and wherein R is cyclopentyl or cyclohexyl. A much preferred titanium compound is titanium isopropoxy heptacyclopentyl silsesquioxane.

Another class of suitable titanium compounds is formed by compounds of the general formula

Ti(OR')₄ (II)

wherein R' is selected from trialkylsilyl, cycloalkyl, arylalkyl and alkyl having 1 to 9 carbon atoms, suitably 2 to 4 carbon atoms. Preferred compounds are those of the above formula, wherein R' is trimethylsilyl, cyclopentyl, cyclohexyl, benzyl, isopropyl, n-butyl, isobutyl or 2-ethylhexyl. Much preferred titanium compounds are tetra(isopropyl) titanate, tetra(n-butyl) titanate and tetrakis(trimethylsilyl) titanate.

Yet another class of suitable titanium compounds are titanate chelates of the general formula 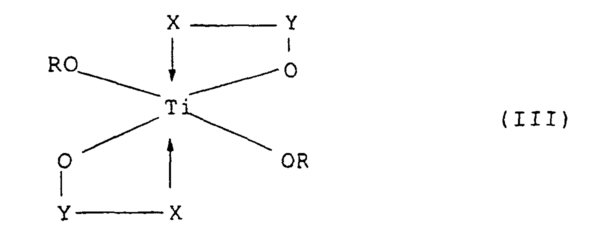 wherein X is a functional group containing oxygen or nitrogen which forms the chelating bond with titanium; Y represents a carbon chain of two or three carbon atoms which may or may not contain a double bond and/or a methyl or ethyl branching and R" represents hydrogen or C1-C3 alkyl. Preferably, X is a carbonyl group-containing moiety, Y is a chain of two carbon atoms with a methyl side chain bonded to the carbonyl group and R" is hydrogen.

Preferred examples of the titanate chelates are acetylacetonate titanate chelate (wherein the X-Y- entity is CH₃C(O)-CH=C(CH₂)-), ethyl acetoacetate titanate chelate (wherein the X-Y- entity is C₂H₅OC(O)-CH=C(CH₃)-) and the ammonium salt of lactic acid titanate chelate (wherein the X-Y- entity is NH₄⁺ -OC(O)-CH(CH₃)- and R" is H). These three compounds are sold by DuPont under the respective trade names Tyzor GBA, Tyzor DC and Tyzor LA (Tyzor is a trademark).

A further suitable titanate chelate is a mixture of chelates sold under the trade name Tyzor TE by DuPont, which mixture comprises at least one component having the structural formula

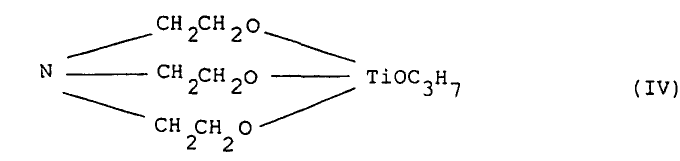

The invention is further illustrated by the following examples without restricting the scope of the invention to these specific embodiments.

The experiments were carried out in a continuous epoxidation bench scale unit containing two vessels on automatic weight balances containing respectively the EBHP and propene feed streams, two high pressure pumps, a fixed bed reactor, a third pump for pumping a recycle stream over the reactor, means to maintain the reactor continuously at temperatures between 60 and 120 °C, a stripper to remove light boiling components like propene, a cooler and a vessel for receiving the product.

The feeds were supplied to the reactor via the two high pressure pumps and mixed together before entering the reactor. The reactor was operated liquid full at 50 bara pressure. A large recycle stream was maintained over the reactor to have isothermal operation of the reactor bed. The feed of propene and a 35 wt% EBHP solution in ethylbenzene was mixed with the recycle stream prior to introduction into the reactor. A compositional analysis of the reaction mixture was carried out by means of Super Critical Fluid Chromatography (SFC).

The following process conditions were maintained during all experiments:

| | |
|---|---|
| throughput EBHP solution | 30 grams/hour |
| throughput propene | 18 grams/hour |
| recycle flow | 2.5 kg/hour |
| temperature | 110 °C. |

### (The EBHP solution used was a 35 wt% EBHP solution in ethylbenzene.)

The soluble titanium compound was added as a 0.05 wt% stock solution in dry ethylbenzene to the reaction mixture prior to introduction into the reactor. The recycle stream was also mixed with the propene/EBHP/ethylbenzene feed prior to introduction into the reactor. In all experiments the titanium compound was dosed in such amount that the titanium level in the reaction mixture was 10 ppmw calculated as metallic Ti and based on total reaction mixture.

The catalyst used in the reactor was a partly deactivated titanium/silica catalyst obtained from the epoxidation section of a commercial styrene/propylene oxide coproduction process.

The activity of the catalyst is expressed as "K85" indicating the reaction rate constant in kg² of liquid per kg of catalyst per mole per hour (kg²/(kg*mole*h)) normalised at 85 °C assuming that second order reaction kinetics apply.

### Example 1

After starting up the continuous epoxidation bench scale unit, the unit was operated under the conditions indicated and without titanium-dosing until a certain conversion level was reached at which the reaction rate remained at a constant level for about 50 hours (approximately from runhour 100 to runhour 150). The reaction rate expressed as K85 was about 3.0 kg²/(kg*mole*h).

Then, after approximately 150 run hours, the dosing of titanium isopropoxy heptacyclopentyl silsesquioxane at a dosing level of 10 ppmw Ti in the feed was started.

The increase in activity of the titanium/silica catalyst is indicated in Table I. K85 is expressed in kg²/(kg*mole*h).

**TABLE I**

| Activation with titanium isopropoxy heptacyclopentyl silsesquioxane | | | | | | |
|---|---|---|---|---|---|---|
| Runhour | 150 | 170 | 190 | 210 | 230 | 250 |
| K85 | 3.0 | 4.7 | 6.0 | 6.6 | 6.6 | 6.6 |

### Example 2

Example 1 was repeated, except that the titanium compound used now was tetrakis(trimethylsilyl) titanate and that titanium dosing was started at runhour 140 after the K85 had been constant at 3.0 kg²/(kg*mole*h) for 50 hours.

The results are indicated in Table II.

**TABLE II**

| Activation with tetrakis(trimethylsilyl) titanate | | | | | | |
|---|---|---|---|---|---|---|
| Runhour | 140 | 150 | 160 | 170 | 190 | 220 |
| K85 | 3.0 | 4.2 | 4.6 | 6.0 | 6.0 | 6.0 |

### Example 3

Example 1 was repeated, except that the titanium compound used now was tetra(isopropyl) titanate and that titanium dosing was started at runhour 110 after the K85 had been constant at approximately 3.2 kg²/(kg*mole*h) for 50 hours.

The results are indicated in Table III.

**TABLE III**

| Activation with tetra(isopropyl) titanate | | | | | | |
|---|---|---|---|---|---|---|
| Runhour | 110 | 120 | 130 | 150 | 170 | 190 |
| K85 | 3.2 | 4.9 | 6.3 | 6.1 | 5.9 | 5.9 |

From the Examples it can be seen that the dosing of a soluble titanium catalyst to the feedstream, which is to be contacted with a partly deactivated heterogeneous titanium-based epoxidation catalyst, results in a remarkable re-activation of the catalyst. In all cases the activity of the catalyst immediately started to increase when dosing of the soluble titanium compound started and levelled off at a constant activity which was about twice as high as the activity of the partly deactivated catalyst.

## Claims

1. Process for the epoxidation of propene into propylene oxide, which process comprises adding an titanium compound to the liquid feed of an epoxidation reaction before this feed is contacted with the titanium containing heterogeneous at least partly deactivated catalyst inside the reactor, wherein the solubility of the titanium compound in the liquid feed is higher than the amount of titanium compound added to this feed.

2. Process according to claim 1, wherein the heterogeneous titanium-containing catalyst is a titanium/silica based catalyst.

3. Process according to claim 1 or 2, wherein the titanium compound is added in such amount that the titanium content (as metal) in the feed entering the epoxidation reactor is in the range of from 1 to 100 ppmw, preferably from 5 to 50 ppmw, based on total feed.

4. Process according to any one of claims 1-3, wherein the titanium compound is a titanasilsesquioxane of the general formula TiLR₇Si₇O₁₂, wherein L is an alkyl, cycloalkyl, arylalkyl, alkylaryl, alkoxy, aryloxy, siloxy, amido or hydroxyl group and R is a cyclopentyl, cyclohexyl or cycloheptyl group.

5. Process according to claim 4,. wherein the titanium compound is titanium isopropoxy heptacyclopentyl silsesquioxane.

6. Process according to any one of claims 1-3, wherein the titanium compound is a compound of the general formula Ti(OR')₄, wherein R' is selected from trimethylsilyl, cycloalkyl and alkyl having 1 to 9 carbon atoms.

7. Process according to claim 6, wherein the titanium compound is tetra(isopropyl) titanate, tetra(n-butyl) titanate or tetrakis(trimethylsilyl) titanate.

8. Process according to any one of claims 1-3, wherein the titanium compound is a titanate chelate of the qeneral formula 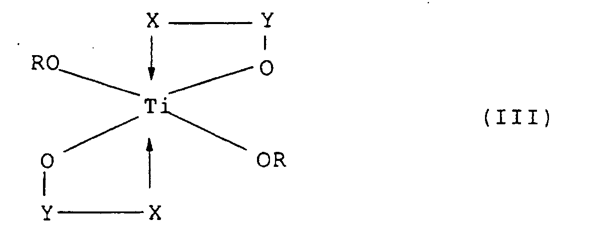 wherein X is a functional group containing oxygen or nitrogen which forms the chelating bond with titanium; Y represents a carbon chain of two or three carbon atoms which may or may not contain a double bond and/or a methyl or ethyl branching and R" represents hydrogen or C1-C3 alkyl.

## Patentansprüche

1. Verfahren zur Epoxidation von Propen zu Propylenoxid, welches Verfahren ein Zusetzen einer Titanverbindung zu der flüssigen Einspeisung einer Epoxidationsreaktion umfaßt, bevor diese Einspeisung mit dem wenigstens teilweise desaktivierten heterogenen titanhältigen Katalysator im Inneren des Reaktors in Kontakt gebracht wird, wobei die Löslichkeit der Titanverbindung in der flüssigen Einspeisung höher ist als die dieser Einspeisung zugesetzte Menge an Titanverbindung.

2. Verfahren nach Anspruch 1, worin der heterogene titanhältige Katalysator ein Titankatalysator auf Siliciumoxidbasis ist.

3. Verfahren nach Anspruch 1 oder 2, worin die Titanverbindung in einer solchen Menge zugesetzt wird, daß der Titangehalt (als Metall) in der in den Epoxidationsreaktor eintretenden Einspeisung im Bereich von 1 bis 100 ppmw, vorzugsweise von 5 bis 50 ppmw, bezogen auf die Gesamteinspeisung, liegt.

4. Verfahren nach einem der Ansprüche 1 bis 3, worin die Titanverbindung ein Titanasilsesquioxan mit der allgemeinen Formel TiLR₇Si₇O₁₂ ist, worin L eine Alkyl-, Cycloalkyl-, Arylalkyl-, Alkylaryl-, Alkoxy-, Aryloxy-, Siloxy-, Amidooder Hydroxylgruppe darstellt und R eine Cyclopentyl-, Cyclohexyl- oder Cycloheptylgruppe darstellt.

5. Verfahren nach Anspruch 4, worin die Titanverbindung Titanium-isopropoxy-heptacyclopentyl-silsesquioxan ist.

6. Verfahren nach einem der Ansprüche 1 bis 3, worin die Titanverbindung eine Verbindung mit der allgemeinen Formel Ti(OR')₄ ist, worin R' unter Trimethylsilyl, Cycloalkyl und Alkyl mit ein bis neun Kohlenstoffatomen ausgewählt ist.

7. Verfahren nach Anspruch 6, worin die Titanverbindung Tetra (isopropyl) titanat, Tetra(n-butyl) titanat oder Tetrakis (trimethylsilyl) titanat ist.

8. Verfahren nach einem der Ansprüche 1 bis 3, worin die Titanverbindung ein Titanatchelat mit der allgemeinen Formel 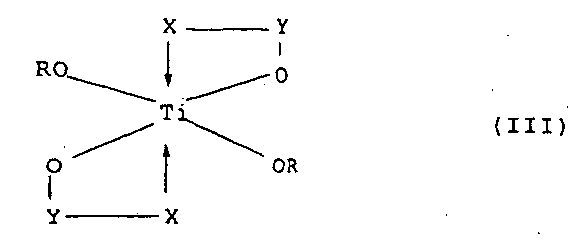 ist, worin X eine Sauerstoff oder Stickstoff enthaltende funktionelle Gruppe ist, die die Chelatbindung mit Titan ausbildet; Y eine Kohlenstoffkette aus zwei oder drei Kohlenstoffatomen darstellt, die eine Doppelbindung und/oder eine Methyl- oder Ethylverzweigung enthalten können oder auch nicht und R" für Wasserstoff oder C₁-C₃-Alkyl steht.

## Revendications

1. Procédé pour l'époxydation de propène en oxyde de propylène, lequel procédé comprend l'addition d'un composé de titane à l'alimentation liquide d'une réaction d'époxydation avant que cette alimentation ne soit mise en contact avec le catalyseur contenant du titane hétérogène au moins partiellement désactivé à l'intérieur du réacteur, dans lequel la solubilité du composé de titane dans l'alimentation liquide est plus élevée que la quantité de composé de titane ajouté à cette alimentation.

2. Procédé suivant la revendication 1, dans lequel le catalyseur contenant du titane hétérogène est un catalyseur à base de titane/silice.

3. Procédé suivant l'un ou l'autre des revendications 1 et 2, dans lequel le composé de titane est ajouté en une quantité telle que la teneur en titane (sous forme métallique) dans l'alimentation entrant dans le réacteur d'époxydation se situe dans la plage de 1 à 100 ppmp, avantageusement de 5 à 50 ppmp, par rapport à l'alimentation totale.

4. Procédé suivant l'une quelconque des revendications 1 à 3, dans lequel le composé de titane est un titanasilsesquioxane de la formule généraleTiR₇Si₇O₁₂, dans laquelle L est un groupe alkyle, cycloalkyle, arylalkyle, alkylaryle, alcoxy, aryloxy, siloxy, amido ou hydroxyle et R est un groupe cyclopentyle, cyclohexyle ou cycloheptyle.

5. Procédé suivant la revendication 4, dans lequel le composé de titane est de l'isopropoxy heptacyclopentyl silsesquioxane de titane.

6. Procédé suivant l'une quelconque des revendications 1 à 3, dans lequel le composé de titane est un composé de la formule générale Ti(OR')₄, dans laquelle R' est choisi parmi les groupes triméthylsilyle, cycloalkyle et alkyle comportant 1 à 9 atomes de carbone.

7. Procédé suivant la revendication 6, dans lequel le composé de titane est du tétra (isopropyl) titanate, tétra (n-butyl) titanate ou tétrakis (triméthylsilyl) titanate.

8. Procédé suivant l'une quelconque des revendications 1 à 3, dans lequel le composé de titane est un chélate de titanate de la formule générale : 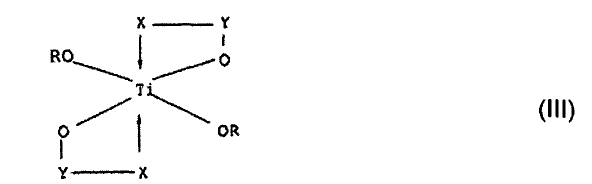 dans laquelle X est un groupe fonctionnel contenant de l'oxygène ou de l'azote qui forme la liaison de chélation avec le titane, Y représente une chaîne carbonée de 2 ou 3 atomes de carbone qui peut ou peut ne pas contenir une double liaison et/ou une ramification méthyle ou éthyle et R représente de l'hydrogène ou un groupe alkyle en C1-C3.
